# EUROPÄISCHE PATENTANMELDUNG

(11) **EP 3 181 988 A1**
(43) Veröffentlichungstag der Anmeldung: **21.06.2017**
(21) Anmeldenummer: 15200486.7
(22) Anmeldetag: 16.12.2015
(51) Int. Cl.: F21K 99/00, G02B 27/09, G02B 19/00, F21V 8/00

(54) **HOMOGENISIERER**

(71) Anmelder: Ivoclar Vivadent AG, 9494 Schaan (LI)
(72) Erfinder: Senn, Bruno, 9056 Gais (CH); Pauler, Markus, 6800 Feldkirch (AT)
(74) Vertreter: Baronetzky, Klaus

(57) **Zusammenfassung**

Es ist ein Homogenisierer, mit einer Eingangsfläche und einer Ausgangsfläche, vorgesehen die zueinander inkongruent sind, wobei sich zwischen der Eingangsfläche und der Ausgangsfläche mindestens eine Schrägfläche erstreckt. Die Schrägfläche weist eine Riffelung auft. Es ist vorgesehen, dass der Homogenisierer als Hohlkörper ausgebildet ist.

## Beschreibung

Die Erfindung betrifft einen Homogenisierer, gemäß dem Oberbegriff von Anspruch 1, sowie ein Lichthärtgerät, gemäß dem Oberbegriff von Anspruch 16.

Es ist bereits seit langem bekannt, bei Lichthärtgeräten, insbesondere zu Dentalzwecken, einen Lichtleiter zu verwenden, um das aus der Lichtquelle stammende Licht dem Behandlungsort zuzuleiten. Der Lichtleiter kann als Lichtleitstab ausgebildet sein, oder als Lichtfaserstab, oder auch in Kombination dieser.

Typischerweise werden als Lichtquellen LED-Chips, aber auch Laser-Chips, verwendet, die je bei einem vorgegebenen Wellenlängenspektrum emittieren, und in dem dentalem Restaurationsmaterial, das gehärtet werden soll, den je eingemischten Photoinitiator, beispielsweise Campherchinon, anregen, um die Polymerisation sicherzustellen.

Es ist ausgesprochen wichtig, dass die Polymerisation gleichmäßig und auch mit entsprechender Tiefenwirkung erfolgt. Während bei mehreren Millimeter tiefen dentalen Restaurationsteilen eine entsprechend längere Polymerisationszeit gewählt wird, um auch die Polymerisation der tieferliegenden Bereiche sicherzustellen, ist es wichtig, dass auch in horizontaler Richtung, also in Richtung quer zur Emissionsachse des Lichtleitstabs, die Polymerisation gleichmäßig vonstatten geht. Typischerweise wird das von der Lichtquelle emittierte Licht gesammelt, wie es beispielsweise aus der US 4 742 432 A1 bekannt ist. Hierdurch entsteht eine Mittenbetonung und Fokussierung auch des emittierten Lichtstrahls. Diese Mittenbetonung ist mit bloßem Auge nicht ohne weiteres zu erkennen, und der Zahnarzt verlässt sich darauf, dass über den gesamten Emissionsbereich mit beispielsweise einem Kreisdurchmesser von 1 cm eine gleichmäßige Lichtemission erfolgt, auch wenn dies aufgrund der Mittenbetonung nicht der Fall sein sollte.

Um der Mittenbetonung und ungleichmäßigen Ausleuchtung entgegenzuwirken, ist es seit langem bekannt geworden, Mischkörper zwischen den Lichtleitstab oder Lichtfaserstab einerseits und der Lichtquelle andererseits anzuordnen. Als Lichtmischkörper werden beispielsweise Glaskörper mit Minireflexkörpern, die in den Glaskörpern gleichmäßig verteilt sind, verwendet. Es kann auch eine Eintrübung des Glaskörpers oder sonstigen Transparentkörpers verwendet werden, um eine Vergleichmäßigung und Diffusion zu gewährleisten.

Ferner ist es bereits vorgeschlagen worden, an dem Lichtmischkörper außen Rippen anzuordnen, die sich im wesentlichen in Längsrichtung des Lichtmischkörpers, also im wesentlichen parallel zur Lichtrichtung erstrecken. Diese Längsrippen erzeugen je nach dem Rippenwinkel unterschiedliche Reflexionen, insbesondere aufgrund des unterschiedlichen Brechungsindexes zwischen der umgebenden Luft und dem aus Glas oder sonstigen transparenten Kunststoff bestehenden Lichtmischkörper.

Es ist auch bereits vorgeschlagen worden, die Rippen des Glas-Lichtmischkörpers zu verspiegeln, indem eine Spiegeischicht aufgedampft wird, und so die Reflektionswirkung zu verbessern.

Die LED-Chips liegen typischerweise in einer Anordnung vor, die rechteckige Aussenabmessungen aufweist, oder sogar quadratisch ist.

Andererseits können viele verschiedenartige Formen der LED-Chip-Anordnung je nach erwünschter Leistung und Auslegung des Lichthärtgeräts zum Einsatz gelangen, so dass zur optimalen Lichtdurchmischung unterschiedliche Lichtmischkörper bereitgehalten werden müssen.

Zudem bestehen Lichtleitstäbe mit 8mm Durchmesser und mit 10mm Durchmesser. Dementsprechend muss auch ausgangsseitig dem Erfordernis Rechnung getragen werden, unterschiedliche Lichtaustrittsflächen bereitzustellen, um eine verlustlose Lichtleitung zu gewährleisten, was die Anzahl der erforderlichen Lichtmischkörper weiter verdoppelt.

Daher liegt der Erfindung die Aufgabe zugrunde, einen Homogenisierer bzw. ein Lichthärtgerät gemäß dem Oberbegriff von Anspruch 1 bzw. 16 zu schaffen, das hinsichtlich der Lichtausbeute und Vergleichmäßigung der Lichtabgabe einerseits, aber auch hinsichtlich der Lagerhaltungskosten andererseits optimiert ist.

Diese Aufgabe wird erfindungsgemäß durch Anspruch 1 bzw. 16 gelöst. Vorteilhafte Weiterbildungen ergeben sich aus den Unteransprüchen.

Erfindungsgemäß ist der Lichtmischkörper oder Homogenisierer mit einer Riffelung ausgestattet, die sich in an sich bekannter Weise von der Eingangsfläche zur Ausgangsfläche erstreckt. Der erfindungsgemäße Homogenisierer ist als Hohlkörper ausgebildet. Im Inneren befindet sich Luft oder ein anderes Gas oder bei Bedarf auch ein Vakuum. Bevorzugt ist der Homogenisierer an der Eingangsfläche und der Ausgangsfläche mit einer transparenten Scheibe verschlossen. Der Innenraum des Hohlkörpers ist insofern abgedichtet, was verhindert, dass sich Verschmutzungen einstellen können.

Erfindungsgemäß liegt der Homogenisierer als Körper mit einer Riffelung vor, die sich um den Außenumfang erstreckt. Die Wand des Hohlkörpers ist insofern nicht gerade, sondern um den gesamten Umfang herum geriffelt. Dies kommt der Verformbarkeit zugute, so dass sich überraschend mit dem erfindungsgemäßen Hohlkörper sämtliche in der Praxis vorkommenden eingangs- und ausgangsseitigen Konfigurationen abdecken lassen.

Um eine Durchmesserreduktion von 10mm auf 8mm an der Ausgangsfläche zu realisieren, wird der Hohlkörper mit seiner geriffelten Wand kurzerhand in die entsprechende Aufnahmebuchse für den 8mm-Lichtleitstab, der sich von dort weg erstreckt, eingeschoben. Die geriffelte Wand ist so elastisch, dass sie sich anpasst und auch zugleich aufgrund ihrer Elastizität die Gleichmäßigkeit der Riffelung beibehält. Der Rippenwinkel ist dann etwas steiler, was jedoch der Homogenisierungswirkung nicht entgegensteht.

Auch eingangsseitig lässt sich die Wand des Homogenisier-Hohlkörpers erfindungsgemäß bei Bedarf verformen, so dass auch bei unterschiedlicher Bestückung der vorbereiteten LED-Chips des Lichthärtgerätes je eine optimale Anpassung realisierbar ist.

Zudem stellt sich automatisch bei der erfindungsgemäßen Ausgestaltung des Hohlkörpers ein Übergang von rechteckig auf rund ein. Typischerweise ist die Eingangsfläche rechteckig oder mehreckig und die Ausgangsfläche rund. Dies bedingt automatisch eine Schrägstellung der Wände des Hohlkörpers, was ebenfalls der Multireflektion und der Durchmischung des eingeleiteten Lichts zugute kommt.

Soweit Sammellinsen insbesondere eingangsseitig des Homogenisierers vorgesehen sind, werden diese beispielsweise als plan-konvexe Linsen realisiert und können bei Bedarf auch gleich die Eingangsseite des Homogenisierers abdecken.

Abgesehen von der gleichsam automatischen Formanpassung an der Eingangsseite und der Ausgangsseite des erfindungsgemäßen Hohlkörpers ist es günstig, dass durch die Riffelung mit dem gewinkelten Rippen eine nahezu verlustlose oder zumindest verlustarme Homogenisierung der eingeleiteten Lichtstrahlung stattfindet.

Dass eingeleitete Licht wird durch die schrägen Winkel, in denen es auftrifft und reflektiert wird stets mehrfach reflektiert, was der Streuwirkung zugute kommt.

In einer weiteren bevorzugten Ausgestaltung ist es vorgesehen, den Hohlkörper aus zwei Halbschalen auszubilden. Diese können über eine Rastverbindung miteinander verbunden sein, so dass sie einen im Querschnitt ringförmigen hohlen Reflektor bilden.

Anstelle dessen kann der erfindungsgemäße Rillenreflektor auch aus einer beliebigen Mehrzahl von Segmenten zusammengesetzt sein. Bevorzugt ist der Reflektor aus Polycarbonat mit einer hohen Oberflächengüte und weist eine Beschichtung mit einer reflektierenden Aluminiumschicht sowie einer zusätzlichen Schutzschicht auf dieser auf.

Die Eingangsseite oder Eingangsfläche des reflektierenden Hohlkörpers kann eine beliebige Form, in Anpassung an die verwendeten - und gegebenenfalls vergossenen - LED-Chips haben. Typischerweise ist die Hüllkurve der vergossenen LED-Chips mindestens mehreckig, und der Hohlkörper kann sich dort eng an die in SMD-Technik aufgebrachten vergossenen Chips anlehnen. Typischerweise bildet die Vergussmasse dann je eine Sammellinse.

Die voneinander beabstandeten LED-Chips weisen mindestens teilweise unterschiedliche Emissionsmaxima auf. Mindestens teilweise sind sie nicht koaxial zur optischen Achse der Eingangsseite des Lichtleitstabs angeordnet. Dies bedingt eine asymmetrische Einleitung von Licht in mindestens einer Farbe in den Homogenisierer. Aufgrund der Multireflektion an den in mehreren Achsen schrägen Rippen des erfindungsgemäßen reflektierenden Hohlkörpers wandert der emittierte Lichtstrahl mehrfach reflektiert in dem Hohlkörper hin und her, bis er den Hohlkörper verlässt.

Insofern ist es günstig, dass der erfindungsgemäße Homogenisierer als innen reflektierender Hohlkörper ausgebildet ist.

Während bei einem fest montierten Lichtleitstab eine zusätzliche Abdeckscheibe ausgangsseitig des Homogenisierers entbehrlich ist, ist es günstig, eine transparente Abdeckscheibe vorzusehen, insbesondere, wenn der Lichtleitstab austauschbar ist. Es ist auch möglich, eine in Maßen nachgiebige Kupplung zu realisieren, die dann sowohl für 8mm-Lichleitstäbe als auch für 10mm-Lichtleitstäbe passt.

Die Rippen der Riffelung können einen beliebigen geeigneten Verlauf aufweisen. Als besonders günstig hat sich eine Sägezahn-Form oder eine Dreiecks-Form des Profils der Rippen in der Abwicklung herausgestellt. Es kann auch günstig sein, die Rippen oder Riffelung nicht mit einer regelmäßigen Form, beispielsweise einer Dreiecks-Form oder einer Sinus-Form mit konstanter Periode, zu realisieren, sondern unregelmäßig, so dass auftreffende Lichtstrahlen in beliebiger und undefinierter Weise reflektiert werden.

Während es bevorzugt ist, die Rippen in konstanter Weise von der Eingangsfläche zur Ausgangsfläche verlaufen zu lassen, kann es in einer modifizierten Ausgestaltung auch günstig sein, die Höhe und/oder die Breite mindestens eines Teils der Rippen über den Verlauf von der Eingangsfläche zur Ausgangsfläche zu ändern.

Gemäß einer vorteilhaften Ausgestaltung der Erfindung ist es vorgesehen, dass die Umfangsstrecke der Eingangsfläche und der Ausgangsfläche gleich ist.

Gemäß einer vorteilhaften Ausgestaltung der Erfindung ist es vorgesehen, dass Rippen der Riffelung sich durchgängig von der Eingangsfläche zur Ausgangsfläche erstrecken.

Gemäß einer vorteilhaften Ausgestaltung der Erfindung ist es vorgesehen, dass die Rippen der Riffelung radial einwärts des Homogenisierers betrachtet spitz zulaufen.

Gemäß einer vorteilhaften Ausgestaltung der Erfindung ist es vorgesehen, dass Rippen der Riffelung radial einwärts betrachtet abgerundet zulaufen, insbesondere mit einem Radius von weniger als einem Drittel, bevorzugt etwa einem Sechstel ihres Abstandes voneinander und/oder ihrer Höhe.

Gemäß einer vorteilhaften Ausgestaltung der Erfindung ist es vorgesehen, dass Rippen Schrägflächen aufweisen, und die Schrägflächen einander benachbarter Rippen zueinander einen Winkel zwischen 60 und 110 Grad, bevorzugt über 80 Grad aufweisen.

Gemäß einer vorteilhaften Ausgestaltung der Erfindung ist es vorgesehen, dass die Rippen (20) im Schnitt betrachtet einen im wesentlichen sinusförmigen Verlauf aufweisen.

Gemäß einer vorteilhaften Ausgestaltung der Erfindung ist es vorgesehen, dass die Riffelung (18) an einer Folie ausgebildet ist, die biegsam ausgebildet ist und an die gewünschte Form der Eingangsfläche (12) einerseits und der Ausgangsfläche (22) andererseits angepasst ist.

Weitere Vorteile, Einzelheiten und Merkmale ergeben sich aus der nachfolgende Beschreibung der mehrere Ausführungsbeispiele der Erfindung anhand der Zeichnung.

Es zeigen:
- Fig. 1: eine schematische Anordnung eines aufgeschnittenen erfindungsgemäßen Homogenisierers mit benachbart angeordneten LED-Chips;
- Fig. 2: eine andere Ausführungsform des Homogenisierers gemäß der Erfindung, wobei die LED-Chips sich in dem Homogenisierer befinden;
- Fig. 3: einen Schnitt gemäß der Ausführungsform Fig. 2;
- Fig. 4: eine weitere Ansicht der Ausführungsform gemäß Fig. 2 und 3;
- Fig. 5A bis 5D: verschiedene mögliche LED-Anordnungen zur Verwendung mit einem erfindungsgemäßen Homogenisierers; und
- Fig. 6A bis 6C: mögliche Formen des Innenreflektors des erfindungsgemäßen Homogenisierers.

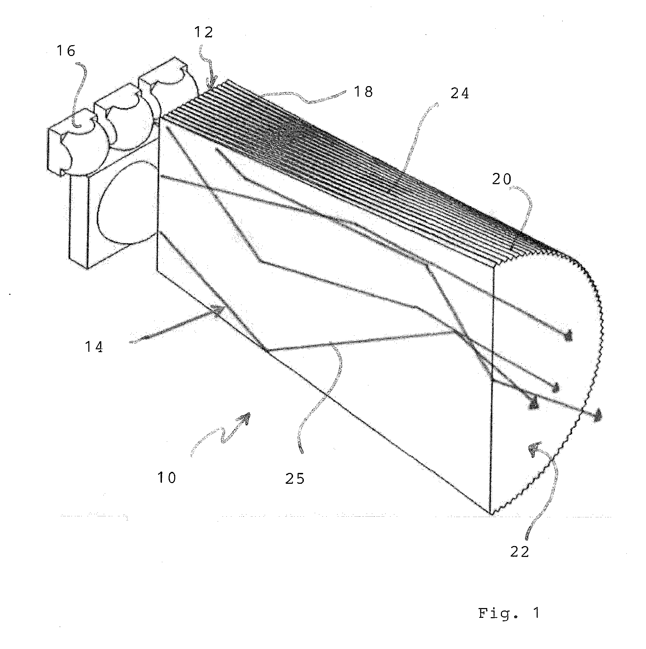

Aus Fig. 1 ist ein erfindungsgemäßer Homogenisierer 10 als Teil eines erfindungsgemäßen Lichthärtgeräts ersichtlich. Einer Eingangsfläche 12 des Homogenisierers 10 benachbart ist eine Mehrzahl von LED-Chips 14 angeordnet, die je einzeln mit einer transparenten Vergussmasse 116 unter Bildung von Sammellinsen vergossen sind. In diesem Ausführungsbeispiel sind die vergossenen LED-Chips 14 von der Eingangsfläche beabstandet. Die Eingangsfläche 12 ist etwa quadratisch, wobei in Fig. 1 eine Rechteck-Form dargestellt ist, nachdem der Homogenisierer 10 aufgeschnitten dargestellt ist.

Der Homogenisierer 10 ist als Hohlkörper ausgebildet und weist an seinem Außenumfang eine Riffelung 18 auf. Die Riffelung 18 besteht aus einer Vielzahl von Rippen 20, die gleichmäßig um den Umfang verteilt sind. Die Rippen 20 erstrecken sich im Verlauf des Homogenisierers an dem Außenumfang entlang, also von der Eingangsfläche 12 zu einer Ausgangsfläche 22.

Die Rippen 20 sind im dargestellten Ausführungsbeispiel als Dreiecksrippen ausgebildet. Das gesamte die Wand 24 des Homogenisierers 10 bildende Material ist geriffelt, die Wand 22 ist insofern innen als auch außen geriffelt.

Die Wand 24 ist zudem innen mit einer Verspiegelung versehen. Die Verspiegelung kann beispielsweise durch eine aufgedampfte Aluminiumschicht realisiert sein. Auf diese ist dann bevorzugt noch eine Schutzschicht aufgebracht, um gleichbleibend gute Reflektionseigenschaften zu gewährleisten.

Der Hohlkörper oder Homogenisierer 10 besteht mit seiner Wand 24 aus Polycarbonat mit mindestens innen einer hohen Oberflächengüte und geringer Rauigkeit.

Die Ausgangsfläche 22 ist in den dargestellten Ausführungsbeispiel kreisrund. Sie ist geeignet, Licht dem Eingangsanschluss eines nicht dargestellten Lichtleiters zuzuführen. Der Eingangsanschluss des Lichtleiters hat mit höchstens wenigen Prozent Abweichungen exakt die gleiche Fläche wie die Ausgangsfläche 22. Damit ist ein praktisch vollständiger Lichtübergang vom Homogenisierer 10 zum Lichtleiter gewährleistet.

In Fig. 1 sind schematisch Lichtstrahlen 25 dargestellt, die mehrfach reflektiert durch den Homogenisierer 10 verlaufen. Besonders wichtig ist die schräge Reflektion an den Rippen 20 innen, die zu einem asymetrischen und insofern unkontrollierten Verlauf der Lichtstrahlen 25 führt und die erwünschte Homogenisierung bereitstellt.

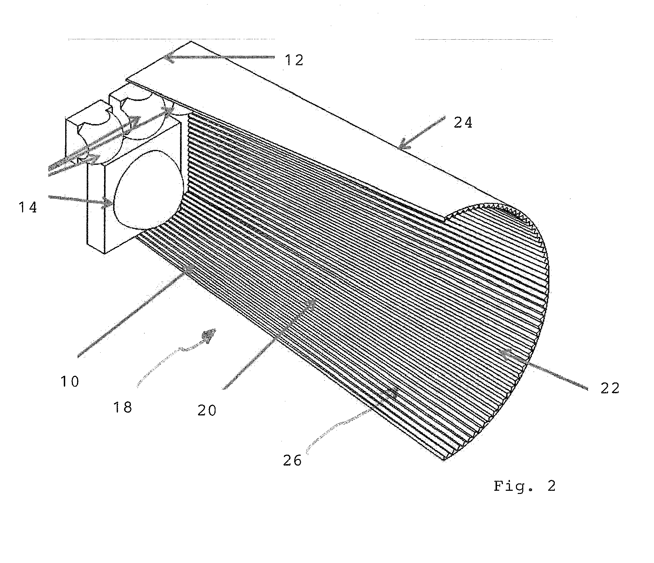

In Fig. 2 ist eine weitere Ausführungsform eines erfindungsgemäßen Homogenisierers dargestellt. Bei dieser Ausführungsform ist die Eingangsfläche 12 als rechteckige Hüllkurve für die LED-Chips 14 ausgebildet. Diese sind in dem Homogenisierer 10 aufgenommen. Mit dieser Ausgestaltung ist sichergestellt, dass sämtliches emittierte Licht gleich in den Homogenisierer 10 gerät und die große Chance hat, an den Rippen 20 reflektiert und asymmetrisch umgelenkt zu werden.

Bei der in Fig. 2 dargestellten Ausführungsform ist die Wand 24 aus einer gebogenen und als Rohr verschweißten Folie aus Polycarbonat realisiert. Außen ist die Wand 24 glatt, während sie innen einen geriffelten Reflektor bildet.

Die Ausgangsfläche 22 ist wiederum kreisrund. Sie weist die gleiche Umfangslänge wie die Eingangsfläche 12 auf. Durch die Forminkongruenz entstehen schräge Seitenflächen des Reflektors 26, zusätzlich zur Schräge der Rippen 20. Die mehrachsige Schrägheit des Reflektors 26 und der Riffelung 18 trägt zur Verbesserung des Homogenisierergebnisses bei.

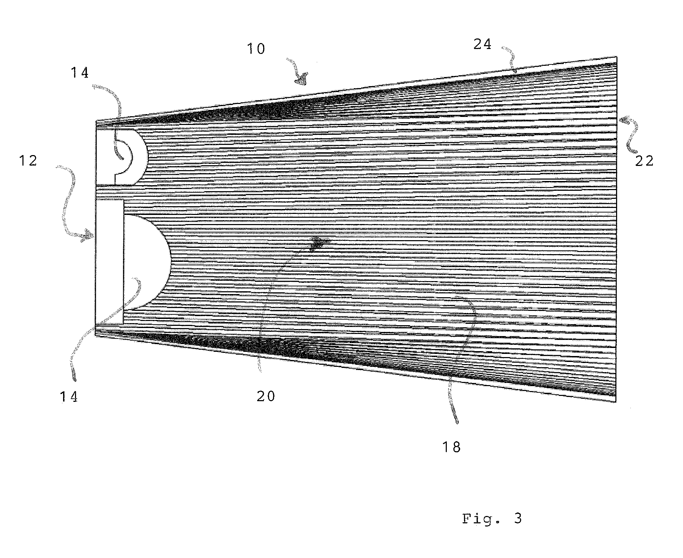

Die Ausführungsform des Homogenisierers gemäß Fig. 2 ist in Fig. 3 im Schnitt dargestellt. Gleiche Bezugszeichen weisen hier wie auch in den weiteren Figuren auf gleiche oder entsprechende Teile hin. Aus Fig. 3 ist die Erstreckung der Riffelung senkrecht sowohl zur Eingangsfläche 12 als auch zur Ausgangsfläche 22 deutlich ersichtlich. Es ist auch ersichtlich, dass die umlaufende Wand 24 schräg verläuft und insofern die Eingangsfläche an die Ausgangsfläche anpasst.

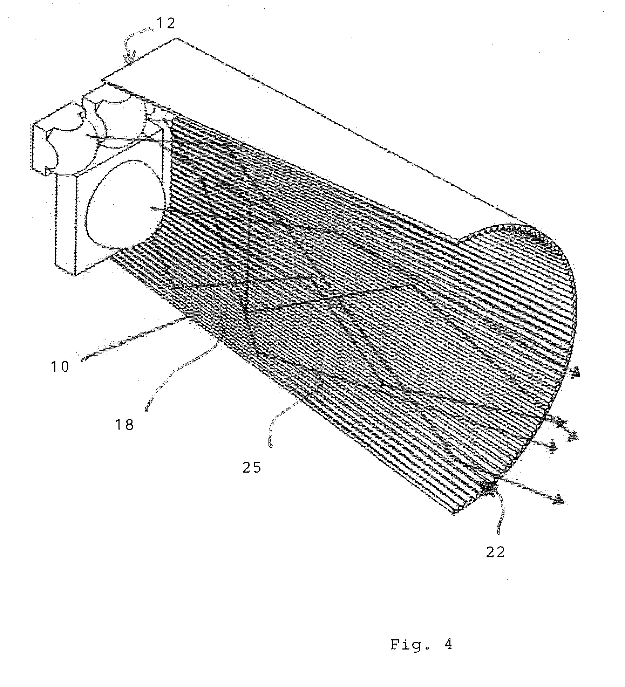

Die in Fig. 4 dargestellte Ausführungsform entspricht der in Fig. 2 dargestellten Ausführungsform. Zusätzlich ist ersichtlich, in welcher Weise die Lichtstrahlen 25 mehrfach reflektiert werden und sich von der Eingangsfläche 12 in Richtung zur Ausgangsfläche 22 unter mehrfacher Umlenkung unter Reflexion an der Riffelung 18 erstrecken.

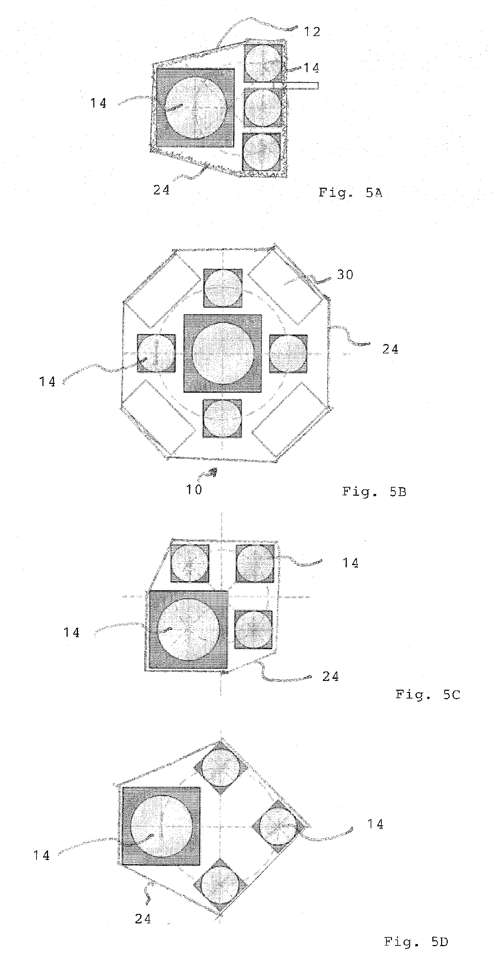

Die Fig. 5A bis 5D zeigen verschiedene mögliche Formen der Eingangsfläche 12, die die LED-Chips 14 einschließt. Die Wand 24 und damit der reflektierende Hohlkörper 10 erstreckt sich je an geraden Außenseiten der vergossenen LED-Chips14 entlang. Zwischen einander benachbarten, jedoch gegeneinander versetzten Ecken verläuft er schräg, so dass die Hüllkurve um die vergossenen LED-Chips diesen eng benachbart verläuft.

Aus Fig. 5A ist eine Anordnung von drei LED-Chips 14 mit gleicher Wellenlänge und in einer Reihe ersichtlich. Von diesen beabstandet, jedoch zu ihnen mitten-symmetrisch angeordnet, ist ein weiterer, größerer vergossener LED-Chip vorgesehen, der ebenfalls von der Wand 24 umgeben ist.

Insofern hat die Wand 24 gemäß Fig. 5A eine sechseckige Form.

Fig. 5B zeigt eine Anordnung von LED-Chips 14, die vergossen sind, und zusätzlich am Außenumfang in den Lücken zwischen diesen verteilten Sensoren 30. Die Sensoren 30 dienen in an sich bekannter Weise zur Erfassung von Lichtstrahlen, die von der Oberfläche des beaufschlagten Materials, beispielsweise von Dentalmaterial, reflektiert werden. In der dargestellten Ausführungsform sind die Sensoren 30 ebenfalls von der Wand 24 umgeben.

In einer modifizierten Ausgestaltung ist es vorgesehen, die Sensoren 30 von der Homogenisierung auszunehmen, und die Wand 24 innerhalb der Sensoren, jedoch außerhalb der LED-CHips 14, verlaufen zu lassen. An den Stellen, an denen sich die Sensoren 30 befinden, kann die Wand 24 dann etwas eingebuchtet sein.

Diese Ausgestaltung ist besonders dann günstig, wenn während der Lichtemission der LED-Chips, also gleichzeitig, und nicht in Impulspausen das reflektierte Licht erfasst werden soll.

Aus Fig. 5C ist eine asymetrische Anordnung der LED-Chips ersichtlich. Drei LED-Chips 14 erstrecken sich seitlich neben bzw. diagonal versetzt gegenüber einem größeren vergossenen LED-Chip 14. Die Wand 24 des Homogenisierers 10 erstreckt sich wiederum in einer möglichst kurzen Umschlingung um die LED-Chips 14.

Aus Fig. 5D ist eine weitere Ausgestaltung der Anordnung der LED-Chips 14 ersichtlich. Drei LED-Chips 14 mit eine kleineren Vergussmasse erstrecken sich im Dreieck, und der Hypotenuse benachbart erstreckt sich ein LED-Chip 14 mit einer größeren Vergussmasse.

Hier ergibt sich eine fünfeckige Anordnung der Wand 24, die sämtliche LED-Chips 14 umgibt.

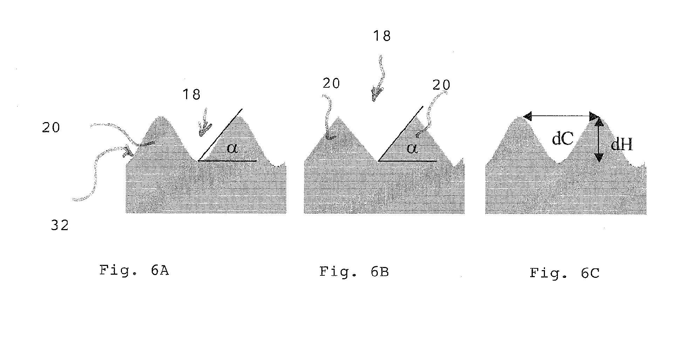

Aus Fig. 6 sind zwei bespielhaft mögliche Formen der Riffelung 18 ersichtlich. Gemäß Fig. 6A verläuft die Riffelung - und damit die Rippen 20 - im wesentlichen sinusförmig. Eine Flanke 32 der Rippe 20 hat einen Schrägstellungswinkel α von etwa 45 Grad. Dieser Winkel ergibt sich zugleich aus dem Verhältnis von dC und dH, also der Periode und der Rippenhöhe gemäß Fig. 6. Beide Größen können konstant sein, oder sich um den Umfang der innen liegenden Riffelung 18 ändern.

Während Fig. 6A und Fig. 6C sinusförmige Riffelungen zeigen, ist gemäß Fig. 6B eine dreiecksförmige Riffelung, wiederum mit dem Schrägstellungswinkel α ersichtlich. Der Winkel α kann in weiten Bereichen an die Erfordernisse angepasst werden. Im dargestellten Ausführungsbeispiel liegt er bei 50 Grad, so dass der Öffnungswinkel der Rippen 80 Grad beträgt. Eine weitere beispielhafte Größe ist α = 20 Grad, entsprechend einem Öffnungswinkel von 140 Grad.

## Patentansprüche

1. Homogenisierer, mit einer Eingangsfläche und einer Ausgangsfläche, die zueinander insbesondere inkongruent sind, wobei sich zwischen Eingangsfläche (12) und Ausgangsfläche (22) mindestens eine Schrägfläche erstreckt, die eine Riffelung (18) aufweist, **dadurch gekennzeichnet, dass** der Homogenisierer (10) als innen reflektierender Hohlkörper ausgebildet ist.

2. Homogenisierer nach Anspruch 1, **dadurch gekennzeichnet, dass** die Eingangsfläche (12) eckig ausgebildet ist, insbesondere viereckig und besonders bevorzugt recht-eckig.

3. Homogenisierer nach Anspruch 1 oder 2, **dadurch gekennzeichnet, dass** die Ausgangsfläche (22) rund ausgebildet ist, bevorzugt kreisrund.

4. Homogenisierer nach einem der vorhergehenden Ansprüche, **dadurch gekennzeichnet, dass** der Eingangsfläche (12) benachbart eine Mehrzahl von LED-Chips (14) angeordnet sind, die insbesondere je mit Sammellinsen vergossen sind.

5. Homogenisierer nach einem der vorhergehenden Ansprüche, **dadurch gekennzeichnet, dass** die Ausgangsfläche (22) verschlossen ist, insbesondere mit einer transparenten Abdeckscheibe.

6. Homogenisierer nach Anspruch 5, **dadurch gekennzeichnet, dass** die Abdeckscheibe in den Homogenisierer (10) eingesetzt ist und an ihrem der Ausgangsfläche (22) zugewandten Wand (24) umfangseitig mit einem insbesondere transparenten Klebstoff mit dem Homogenisierer (10) verklebt ist.

7. Homogenisierer nach Anspruch 5, **dadurch gekennzeichnet, dass** die Abdeckscheibe stumpf auf die Ausgangsfläche (22) des Homogenisierers (10) aufgesetzt und dort insbesondere fixiert ist.

8. Homogenisierer nach einem der vorhergehenden Ansprüche, **dadurch gekennzeichnet, dass** die Ausgangsfläche (22) des Homogenisierers (10) einem Eingangangschluss eines Lichtleiters benachbart ist und insbesondere diese beiden Flächen miteinander fluchten.

9. Homogenisierer nach einem der vorhergehenden Ansprüche, **dadurch gekennzeichnet, dass** die Riffelung (18) eine Vielzahl von Rippen (20) aufweist, die sich um den Innenumfang des Homogenisierers (10) erstrecken, insbesondere zwischen 10 und 100 Rippen (20) und besonders bevorzugt etwa 20 Rippen (20).

10. Homogenisierer nach einem der vorhergehenden Ansprüche, **dadurch gekennzeichnet, dass** Rippen (20) der Riffelung (18) sich mit einer gleichbleibenden Form zwischen der Eingangsfläche (12) und der Ausgangsfläche (22) erstrecken.

11. Homogenisierer nach einem der vorhergehenden Ansprüche, **dadurch gekennzeichnet, dass** Rippen (20) der Riffelung (18) in einem regelmäßigen Muster, insbesondere im gleichen Abstand zueinander angeordnet sind.

12. Homogenisierer nach Anspruch 1 bis 11, **dadurch gekennzeichnet, dass** Rippen (20) der Riffelung (18) mit sich im wesentlichen parallel zueinander, jedoch mit voneinander verschiedenen Formen erstrecken.

13. Homogenisierer nach einem der vorhergehenden Ansprüche, **dadurch gekennzeichnet, dass** die Form der Riffelung (18), insbesondere deren Rippen (20), an die von dem Homogenisierer (10) übertragene Lichtwellenlänge oder Lichtwellenlängebereiche angepasst ist.

14. Homogenisierer nach einem der vorhergehenden Ansprüche, **dadurch gekennzeichnet, dass** die Riffelung (18) des Homogenisierers (10) eine Reflexionsschicht aufweist, insbesondere eine aufgedampfte Reflektionsschicht.

15. Homogenisierer nach einem der vorhergehenden Ansprüche, **dadurch gekennzeichnet, dass** er mehrteilig, insbesondere zweiteilig ausgebildet ist, wobei die Teile des Homogenisierers (10) insbesondere durch Rastverbindungen aneinander gehalten sind.

16. Lichthärtgerät, mit einem Homogenisierer gemäß einem der Ansprüche 1 bis 22, der im Strahlengang einer Lichtquelle nachtgeschaltet ist, und insbesondere einem Lichtleitstab vorgeschaltet ist.
